# EUROPÄISCHE PATENTANMELDUNG

(11) **EP 2 255 779 A2**
(43) Veröffentlichungstag der Anmeldung: **01.12.2010**
(21) Anmeldenummer: 10002261.5
(22) Anmeldetag: 05.03.2010
(51) Int. Cl.: A61K 8/41, A61K 8/73, A61K 8/81, A61Q 5/06, A61Q 5/12

(54) **Haarvolumenstärkendes Mittel**

(30) Priorität: 27.05.2009 DE 102009022786
(71) Anmelder: Beiersdorf Aktiengesellschaft, 20253 Hamburg (DE)
(72) Erfinder: Fey, Sven, 22397 Hamburg (DE); Neuhoff, Henrike, 22359 Hamburg (DE); Wendicke, Silke, 22085 Hamburg (DE)
(74) Vertreter: Porath, Stefan

(57) **Zusammenfassung**

Haarbehandlungszubereitung bestimmt zum Verbleib im Haar enthaltend Polyquaternium-16, Cetrimonium Chlorid, Hydroxypropyl Guar und aufweisend eine mittlere Transmission gemessen mit dem Turbiscan MA 2000 von wenigstens 80%, bevorzugt von wenigstens 85%.

## Beschreibung

Mehr Fülle und Volumen ist für die Verbraucherin eines der wichtigsten Produktanforderungen im Haarpflegesegment, den Schönheit und Gesundheit wird mit vollem Haar verbunden. Der Literatur nach zu urteilen kann der Wunsch nach gepflegtem, glänzenden Haar und einer voluminösen, haltbaren Frisur nur unter Eingehen von Kompromissen erfüllt werden (Kosmetik und Hygiene, Wiley-VCH, 2004, S. 259). Um Volumen zu erzeugen, wird das Haar meist leicht aufgeraut und die Reibung zwischen den einzelnen Haaren wird erhöht. Durch dieses Aufrauen werden allerdings die Haarstruktur und die damit verbundenen Eigenschaften wie Kämmbarkeit, Glanz und Geschmeidigkeit verschlechtert.

WO 2002060397 offenbart eine Zubereitung mit einer alkoholisch-wässrigen Phase mit Fixiermittel, Salz, Emulgator und einer nichtwässrigen Phase mit flüchtigem Silikon oder Kohlenwasserstoff.

WO 1991015186 offenbart eine sehr spezielle Ausspülkonditionierzubereitung mit Konditioniermittel und bestimmten Stylingpolymeren sowie nichtwässrigen Lösungsmitteln.

EP 1479368 offenbart eine transparente nichtemulsionsförmige Haarkonditionierzubereitung mit Polyquaternium 37 und ohne Fettalkohole.

Zur Pflege des Haares gibt es Markt verschiedenste Applikationsformen. Hierbei erfreuen sich Convenience-Produkte, die nach der Anwendung im Haar verbleiben, zunehmender Beliebtheit. Die meistens Produkte, die zur Pflege des Haare im Haar verbleiben, sind milchige Emulsionen. Diese Optik ruft beim Verbraucher oftmals die Befürchtung einer Haarbeschwerung hervor. Die angebotenen transparenten Formulierungen sind Stylingprodukte (Gele), die keinen oder lediglich einen sehr geringen Pflegeeffekt nach Applikation auf das Haar aufweisen.

Es fehlte dem Stand der Technik also an Produkten, die die Nachteile des Standes der Technik nicht aufweisen.

Es hat sich überraschend und für den Fachmann nicht vorhersehbar gezeigt, dass Haarbehandlungszubereitung bestimmt zum Verbleib im Haar enthaltend Polyquaternium-16, Cetrimonium Chlorid, Hydroxypropyl Guar und aufweisend eine mittlere Transmission gemessen mit dem Turbiscan MA 2000 von wenigstens 80%, bevorzugt von wenigstens 85% den Nachteilen des Standes der Technik abhelfen.

Diese transparente Formulierung, die im Haar verbleibt und sowohl eine pflegende als auch eine stylende Wirkung hat, weist ungewöhnlich gute kosmetische Eigenschaften auf. Die Ermittlung des Trübungsgrades der Formel erfolgte durch Messung der mittleren Transmission mit einem Turbiscan MA 2000. Hierbei erreichte das trübste Produkt, die zum Anmeldezeitpunkt in Deutschland käufliche 3WetterTaft Mega Volumen Power Haarverdicker-Lotion von Schwarzkopf (Produkt B), einen mittlere Transmission von 73 %. Die erfindungsgemäße Formulierung (Produkt A) ist mit einer mittleren Transmission von 88 % deutlich klarer.

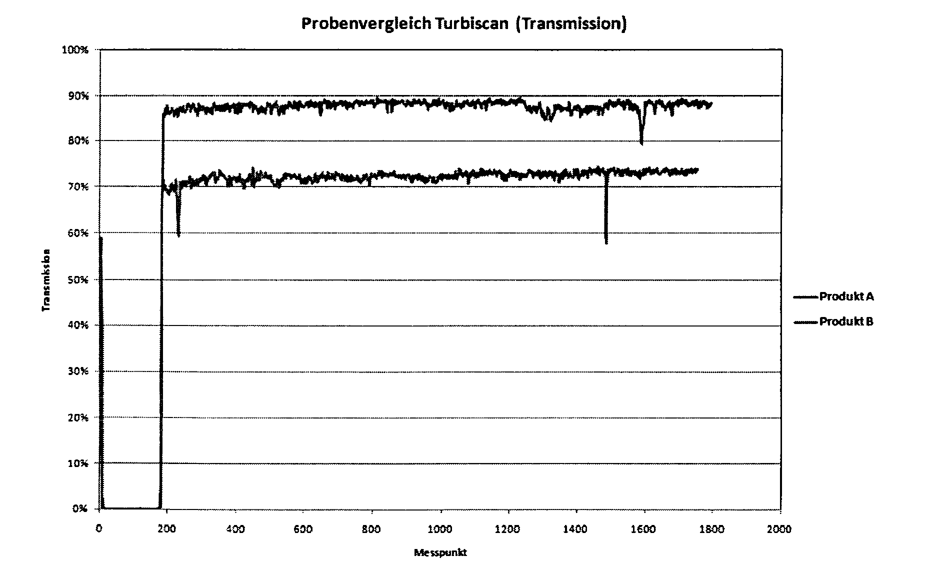

Es hat sich gezeigt, dass der Verbraucher vor der ersten Anwendung des transparenten Produktes mit Skepsis reagiert. Dies ist auf die mangelnde Erfahrung in Bezug auf transparente Leave-In Formulierungen, die keine Stylinggele sind, zurückzuführen. Nach der Applikation waren die Verbraucher von der pflegenden sowie gleichzeitig Volumen gebenden Wirkung positiv überrascht.

Die pflegende Wirkung der transparenten Formulierung wird durch eine Kombination aus Hydroxypropyl Guar, Cetrimoniumchlorid und Jojobaöl erreicht. In einem Test wurde die pflegende Wirkung der Formulierung bestätigt. Eine Kämmkraftmessung zeigt, dass sich die relative Kämmkraft nach Applikation von Produkt A um durchschnittlich 65 % reduziert. Nach Applikation von Produkt B (die zum Anmeldezeitpunkt in Deutschland käufliche 3WetterTaft Mega Volumen Power Haarverdicker-Lotion von Schwarzkopf) wird die relative Kämmkraft lediglich um durchschnittlich 42 % reduziert.

Die Volumen gebende Wirkung der transparenten Formulierung wird durch Polyquaternium-16 erzielt. Die stylende Wirkung wurde ebenfalls in einem Fülle-Volumen-Test bestätigt.

Bevorzugt ist es, wenn zusätzlich 8 bis 20% Ethanol enthalten sind.

Auf den Einsatz von Ethanol kann auch verzichtet werden. Das erfindungsgemäße Produkt kann auch nach der Anwendung gleich wieder ausgespült werden. Nachgewiesenermaßen wirkungsvoll ist aber der Einsatz von Ethanol und der Verbleib des Produktes im Haar.

Weiter ist bevorzugt, wenn Polyquarternium-16 in Aktivkonzentrationen von 0,1 bis 1,5% vorliegt. Weiter ist bevorzugt, wenn Cetrimonium Chloride in Aktivkonzentrationen von 0,05 bis 0,4% vorliegt. Weiter ist bevorzugt, wenn Hydroxypropyl Guar in Aktivkonzentrationen von 1 bis 1,5% vorliegt. Weiter ist bevorzugt, wenn Jojobaöl in Konzentrationen von 0,01 bis 1 % vorliegt. Weiter ist bevorzugt, wenn Salicylsäuresalze in Konzentrationen von 0,01 bis 0,5%, bevorzugt von 0,01 bis 0,2 bezogen auf das Salicylat-Ion vorliegt. Weiter ist bevorzugt, wenn Wasser in Konzentrationen von mehr als 80% vorliegt. Erfindungsgemäß ist auch die Verwendung solcher Zubereitungen zum gleichzeitigen Gestalten der Frisur und zur Pflege des Haares und/oder zum Erhöhen des Volumens der Frisur des Menschen.

Den erfindungsgemäßen Zubereitungen können Lichtfilter, wasserlösliche Silikone, Vitamine, Proteine, Pflanzenextrakte, Wirkstoffe (Anti-Schuppen, Creatine, Carnitine, Q10) hinzugefügt werden.

**Beispiele:**

| | Gew. % | 1 | 2 | 3 | 4 | 5 |
|---|---|---|---|---|---|---|
| 1 | Aqua | 79,452 | 80,962 | 74,22 | 71,66 | 81,041 |
| 2 | Citric Acid | 0,018 | 0,018 | 0,4 | 0,01 | 0,018 |
| 3 | Sodium Benzoate | 0,4 | 0,4 | 0,4 | 0,4 | 0,4 |
| 4 | PEG-12 | | | | 4 | |
| 5 | Benzophenone-4 | 0,03 | 0,02 | 0,03 | 0,05 | 0,03 |
| 6 | Glycerin | | | | 0,869 | |
| 7 | PEG-40 Hydrogenated Castor Oil | 0,8 | 0,8 | 0,8 | 0,8 | 0,8 |
| 8 | Hydroxyethylcellulose | | | | 1 | |
| 9 | Alcohol Denat. | 10 | 10 | 15 | 10 | 10 |
| 10 | PEG-12 Dimethicone | | | | 1 | |
| 11 | Cetrimonium Chloride | 0,3 | 0,4 | 0,3 | 0,3 | 0,3 |
| 12 | Sodium Salicylate | 0,2 | 0,2 | 0,2 | 0,2 | 0,2 |
| 13 | Polyquaternium-47 | | | | 2,5 | |
| 14 | Ceteareth-20 | 0,4 | 0,4 | 0,4 | 0,4 | 0,4 |
| 15 | Hydroxypropyl Guar | 1,3 | 1,2 | 1,3 | 1,3 | 1,3 |
| 16 | Polyquaternium-16 | 5 | 5 | 5 | 5 | 5 |
| 17 | Parfum | 0,5 | 0,5 | 0,5 | 0,5 | 0,5 |
| 18 | Aloe Barbadensis Gel | | | | 0,01 | 0,001 |
| 19 | Niacinamide | | 0,1 | | | |
| 20 | Simmondsia Chinensis Oil | | | | 0,001 | 0,01 |
| 21 | Carnitine | 1 | | 1 | | |
| 22 | Creatine | 0,6 | | 0,45 | | |

### Handelsnamen der Rohstoffe

- 4: Polyglycol 600, Clariant, 100 % Aktivgehalt.
- 10: Dow Corning 193 C Fluid, Dow Corning, 100 % Aktivgehalt.
- 11: CTAC 6025 KC, KCI, 25 % Aktivgehalt.
- 13: Merquat 2001, Nalco, 20 % Aktivgehalt.
- 14: Eumulgin B2, Cognis, 100 % Aktivgehalt.
- 15: Jaguar HP105, Rhodia, 94 % Aktivgehalt.
- 16: Luviquat Style, BASF, 20 % Aktivgehalt.
- 21: Natrulon RC-100, Lonza, 100 % Aktivgehalt.
- 22: Cosmocair C 100, Evonik Goldschmidt, 100 % Aktivgehalt.

| | wt % | 6 | 7 | 8 | 9 | 10 |
|---|---|---|---|---|---|---|
| 1 | Aqua | 81,67 | 81,97 | 80,61 | 80,82 | 84,62 |
| 2 | Citric Acid | 0,1 | 0,1 | 0,15 | 0,25 | 0,25 |
| 3 | Sodium Benzoate | 0,4 | 0,4 | 0,4 | 0,4 | 0,4 |
| 4 | Benzophenone-4 | 0,03 | 0,03 | 0,03 | 0,03 | 0,03 |
| 5 | PEG-40 Hydrogenated Castor Oil | 0,8 | 0,8 | 0,8 | 0,8 | 0,8 |
| 6 | Alcohol Denat. | 10 | 10 | 10 | 10 | 10 |
| 7 | Cetrimonium Chloride | 0,3 | 0,5 | 0,3 | 0,2 | 0,3 |
| 8 | Zinc Pyrithione | 1 | | | | |
| 9 | Zinc Carbonate | 0,5 | | | | |
| 10 | Piroctone Olamine | | 0,5 | | 0,3 | |
| 11 | Climbazole | | 0,5 | 0,3 | | |
| 12 | Laureth-9 | 1,8 | 1,8 | 1 | 1,8 | |
| 13 | Polyquaternium-16 | 1 | 1 | 4 | 3 | 1 |
| 14 | Sodium Salicylate | 0,2 | 0,2 | 0,2 | 0,2 | 0,2 |
| 15 | Ceteareth-20 | 0,4 | 0,4 | 0,4 | 0,4 | 0,4 |
| 16 | Hydroxypropyl Guar | 1,3 | 1,3 | 1,3 | 1,3 | 1,3 |
| 17 | Parfum | 0,5 | 0,5 | 0,5 | 0,5 | 0,5 |
| 18 | Ubiquinone | | | | | 0,1 |
| 19 | Panthenol | | | 0,01 | | 0,1 |

### Handelsnamen der Rohstoffe

- 7: CTAC 6025 KC, KCI, 25 % Aktivgehalt.
- 8: Zinc Pyrithione, Beckmann, 48 % Aktivgehalt.
- 9: Zinc Carbonate basic, Chemos, 97 % Aktivgehalt.
- 10: Octopirox, Clariant, 100 % Aktivgehalt.
- 11: Crinipan AD 600306, Symrise, 100 % Aktivgehalt.
- 12: Genapol LA 090, Clariant, 98 % Aktivgehalt.
- 13: Luviquat Style, BASF, 20 % Aktivgehalt.
- 15: Eumulgin B2, Cognis, 100 % Aktivgehalt.
- 16: Jaguar HP105, Rhodia, 94 % Aktivgehalt.
- 18: Coenzym Q10, Kaneka, 100 % Aktivgehalt.

## Patentansprüche

1. Haarbehandlungszubereitung bestimmt zum Verbleib im Haar enthaltend Polyquaternium-16, Cetrimonium Chlorid, Hydroxypropyl Guar und aufweisend eine mittlere Transmission gemessen mit dem Turbiscan MA 2000 von wenigstens 80%, bevorzugt von wenigstens 85%.

2. Zubereitung nach Anspruch 1 **dadurch gekennzeichnet dass** Polyquarternium-16 in Aktivkonzentrationen von 0,1 bis 1,5% vorliegt.

3. Zubereitung nach einem der vorangehenden Ansprüche **dadurch gekennzeichnet, dass** Cetrimonium Chloride in Aktivkonzentrationen von 0,05 bis 0,4% vorliegt.

4. Zubereitung nach einem der vorangehenden Ansprüche **dadurch gekennzeichnet, dass** Hydroxypropyl Guar in Aktivkonzentrationen von 1 bis 1,5% vorliegt.

5. Zubereitung nach einem der vorangehenden Ansprüche **dadurch gekennzeichnet, dass** Jojobaöl in Konzentrationen von 0,01 bis 1 % vorliegt.

6. Zubereitung nach einem der vorangehenden Ansprüche **dadurch gekennzeichnet, dass** Salicylsäuresalze in Konzentrationen von 0,01 bis 0,5%, bevorzugt von 0,01 bis 0,2 bezogen auf das Salicylat-Ion vorliegt.

7. Zubereitung nach einem der vorangehenden Ansprüche **dadurch gekennzeichnet, dass** zusätzlich 8 bis 20% Ethanol enthalten sind.

8. Zubereitung nach einem der vorangehenden Ansprüche **dadurch gekennzeichnet, dass** Wasser in Konzentrationen von mehr als 80% vorliegt.

9. Verwendung von Zubereitungen nach einem der vorangehenden Ansprüche zum gleichzeitigen Gestalten der Frisur und zur Pflege des Haares.

10. Verwendung von Zubereitungen nach einem der vorangehenden Ansprüche zum Erhöhen des Volumens der Frisur des Menschen.
